(19) Europäisches Patentamt
European Patent Office
Office européen des brevets

(11) Numéro de publication: **0 234 995**
**B1**

(12) **FASCICULE DE BREVET EUROPEEN**

(45) Date de publication du fascicule du brevet: **18.04.90**

(51) Int. Cl.[5]: **C 07 D 215/56,** A 61 K 31/47

(21) Numéro de dépôt: **87400165.4**

(22) Date de dépôt: **23.01.87**

(54) **Dérivés de la quinoléine, leur procédé de préparation, leur application à titre de médicaments et les compositions les renfermant.**

(30) Priorité: **24.01.86 FR 8601401**

(43) Date de publication de la demande:
**02.09.87 Bulletin 87/36**

(45) Mention de la délivrance du brevet:
**18.04.90 Bulletin 90/16**

(84) Etats contractants désignés:
**AT BE CH DE ES FR GB GR IT LI LU NL SE**

(56) Documents cités:
**EP-A-0 070 767**

(73) Titulaire: **SANOFI**
**40, Avenue George V**
**F-75008 Paris (FR)**

(72) Inventeur: **Vernieres, Jean-Claude**
**Rue Sabatier Garat**
**F-31600 Muret (FR)**
Inventeur: **Simiand, Jacques**
**136 Chemin de Lacombe**
**F-31600 Muret (FR)**
Inventeur: **Keane, Peter Eugène**
**10, avenue Wiston Churchill**
**F-31100 Toulouse (FR)**

(74) Mandataire: **Bressand, Georges et al**
**c/o CABINET LAVOIX 2 Place d'Estienne d'Orves**
**F-75441 Paris Cédex 09 (FR)**

**EP 0 234 995 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention est relative à de nouveaux dérivés de la oxo-4 dihydro-1,4 quinoléine carboxamide-3, à leur procédé de préparation, à leur application à titre de médicaments et aux compositions les renfermant.

Les composés de l'invention répondent à la formule générale (I):

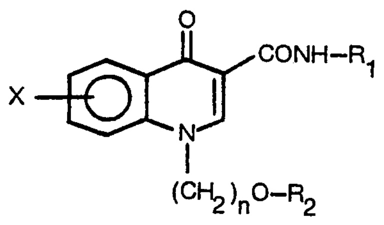

(I)

dans laquelle:

$R_1$ représente un radical alcoyle inférieur linéaire ou ramifié comprenant de 1 à 5 atomes de carbone; un radical alcényle ou alcynyle comprenant de 2 à 5 atomes de carbone; ou un radical cycloalcoyle comprenant de 3 à 5 atomes de carbone,

$R_2$ représente un radical alcoyle comprenant de 1 à 4 atomes de carbone, n est égal à 1 ou 2,

X représente un hydrogène, un halogène, un groupe alcoxy ou alcoyle de 1 à 4 atomes de carbone.

L'invention comprend aussi les sels d'addition avec les acides minéraux ou organiques pharmaceutiquement acceptables.

Les composés, objet de la présente invention, possèdent de très intéressantes propriétés pharmacologiques: ils sont doués, notamment, de remarquables propriétés anticonvulsivante et psychotonique.

L'invention a également pour objet un procédé de préparation des composés de formule (I) caractérisé en ce que l'on fait réagir le composé de formule (II):

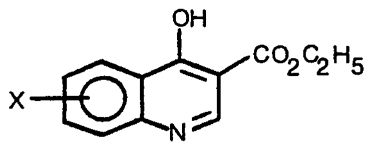

(II)

dans laquelle X a les mêmes significations que dans la formule (I) avec un dérivé halogéné de formule (III):

$$Y—(CH_2)_n—O—R_2 \quad (III)$$

dans lequel Y représente un halogène tel que le chlore ou le brome et n et $R_2$ ont les mêmes valeurs que dans la formule (I) pour obtenir les composés de formule (IV).

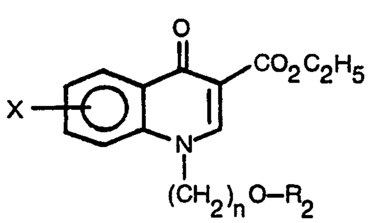

(IV)

sur lequel on fait réagir une amine de formule (V):

$$H_2N—R_1 \quad (V)$$

dans laquelle $R_1$ a les mêmes significations que dans la formule (I) pour obtenir le composé de formule (I).

Les quinoléines de formule (II) sont des composés connus : elles peuvent être préparées selon le procédé décrit par B. RIEGEL et al. (J. Am. Chem., Soc., 1946, *68*, 1264—1266).

Les composés de formule (IV) sont obtenus par chauffage des deux réactifs (II) et (III) à une température comprise entre 60°C et 130°C, en présence d'une base telle que le carbonate de potassium dans un solvant organique tel que le diméthylformamide.

La fixation du radical amine de formule (V) sur le composé de formule (IV) est réalisée par chauffage des réactifs dans un excès de l'amine (V) à une température comprise entre 60°C et 150°C, pendant une durée de 10 h. à 48 h.

La réaction peut être réalisée avantageusement en opérant dans un réacteur métallique fermé de manière à obtenir par chauffage une pression interne de 5 à 50 bars. Dans ces conditions, les réactifs (IV) et (V) sont mis à réagir dans un solvant organique tel que l'alcool éthylique.

Les exemples suivants sont donnés à titre d'illustration de la présente invention.

### Exemple 1

Méthoxyméthyl-1 oxo-4 dihydro-1,4 quinoléine N-propyl carboxamide-3 (I: $R_1 = C_3H_7$; $R_2 = CH_3$; n = 1; X = H); dérivé n° 1 (SR 25776).

a) Méthoxyméthyl-1 oxo-4 dihydro-1,4 quinoléine carboxylate d'éthyl-3 (IV: $R_2 = CH_3$, X = H, u = I)

Un mélange d'hydroxy-4 quinoléine carboxylate d'éthyle-3 (II), 2 g (0.0092 mole de carbonate de potassium, 2 g (0,014 mole) dans 20 ml de diméthylformamide est porté 1 h. à 90°C. Après addition de 0,7 ml (0,0092 mole) de chlorométhyl méthyléther (III) le chauffage est poursuivi pendant 40 h. Après filtration et évaporation du solvant, les cristaux résiduels sont lavés à l'eau et séchés.

Cristaux incolores, rendement: 40%, F = 146°C.

Analyse $C_{14}H_{15}NO_4$

| | | | |
|---|---|---|---|
| Calculé: | C 64,36 | H 5,79 | N 5,36 |
| Trouvé: | C 64,15 | H 5,89 | N 5,17 |

b) Méthoxyméthyl-1 oxo-4 dihydro-1,4 quinoléine N-propyl carboxamide-3

Un mélange de 1 g (0,0038 mole) du produit obtenu dans l'étape précédente et de 28 ml de propylamine est porté pendant 24 h. à 80°C. Après refroidissement, les cristaux sont filtrés, lavés à l'éther isopropylique et séchés sous vide.

Cristaux incolores, rendement: 70%, F = 159°C.

Analyse $C_{15}H_{18}N_2O_3$

| | | | |
|---|---|---|---|
| Calculé: | C 65,68 | H 6,61 | N 10,21 |
| Trouvé: | C 65,96 | H 6,72 | N 10,11 |

### Exemple 2

Méthoxyméthyl-1 oxo-4 dihydro-1,4 quinoléine N-méthyl carboxamine-3 (I: $R_1 = CH_3$, $R_2 = CH_3$, n = 1, X = H) dérivé n° 2 (SR 26004).

Un mélange de méthoxyméthyl-1 oxo-4 dihydro-1,4 quinoléine carboxylate d'éthyle-3 (0,0038 mole) préparé selon l'exemple 1 et de méthylamine à 33% dans l'éthanol (150 ml) est chauffé à 130°C pendant 8 heures dans un réacteur sous une pression de 40 bars. Après évaporation du solvant, le produit attendu est recristallisé dans l'acétate d'éthyle:

Cristaux incolores F = 205°C (Rdt. 37%)

Analyse $C_{13}H_{14}N_2O_3$

| | | | |
|---|---|---|---|
| Calculé: | C 63,40 | H 5,73 | N 11,37 |
| Trouvé: | C 63,38 | H 5,76 | N 11,14 |

### Exemple 3

Méthoxyméthyl-1 oxo-4 dihydro-1,4 quinoléine N-éthyl carboxamide-3 (I: $R_1 = C_2H_5$, $R_2 = CH_3$, N = 1, X = H); dérivé n° 3 (SR 25950).

Ce composé a été préparé en suivant le mode opératoire décrit dans l'exemple 2.

F = 190°C (Rdt. 40%)

Analyse $C_{14}H_{16}N_2O_3$

| | | | |
|---|---|---|---|
| Calculé: | C 64,60 | H 6,20 | N 10,76 |
| Trouvé: | C 64,29 | H 6,10 | N 10,74 |

Les exemples suivants ont été préparés selon le mode opératoire décrit dans l'exemple 1.

### Exemple 4

Méthoxyméthyl-1 oxo-4 dihydro-1,4 quinoléine N-isopropyl carboxamide-3

(I: $R_1$ = 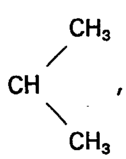 , $R_2 = CH_3$, n = 1, X = H); dérivé n° 4 (SR 25972).

F = 161°C (Rdt. 32%)

Analyse $C_{15}H_{18}N_2O_3$

| | | | |
|---|---|---|---|
| Calculé: | C 65,68 | H 6,61 | N 10,21 |
| Trouvé: | C 65,50 | H 6,69 | N 10,04 |

Exemple 5

Méthoxyméthyl-1 oxo-4 dihydro-1,4 quinoléine N-isobutyl carboxamide-3.

(I: $R_1$ = 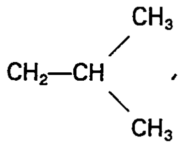 , $R_2 = CH_3$, n = 1, X = H); dérivé n° 5 (SR 25931).

F = 136°C (Rdt. 50%)
Analyse $C_{16}H_{20}N_2O_3$

| | | | |
|---|---|---|---|
| Calculé: | C 66,65 | H 6,93 | N 9,71 |
| Trouvé: | C 66,38 | H 6,96 | N 9,59 |

Exemple 6

Méthoxyméthyl-1 oxo-4 dihydro-1,4 quinoléine N-(propyne-2 yl) carboxamide-3 (I: R = $CH_2—C\equiv CH$, $R_2 = CH_3$, n = 1, X = H) dérivé n° 6 (SR 26080).

F = 213°C (Rdt. 43%)
Analyse $C_{15}H_{14}N_2O_3$

| | | | |
|---|---|---|---|
| Calculé: | C 66,66 | H 5,22 | N 10,36 |
| Trouvé: | C 66,93 | H 5,28 | N 10,31 |

Exemple 7

Méthoxyméthyl-1 oxo-4 dihydro-1,4 quinoléine N-cyclopropyl carboxamide-3.

(I: $R_1$ = 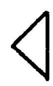 , $R_2 = CH_3$, n = 1, X = H); dérivé n° 7 (SR 26075)

F = 200°C (Rdt. 46%)
Analyse $C_{15}H_{16}N_2O_3$

| | | | |
|---|---|---|---|
| Calculé: | C 66,16 | H 5,92 | N 10,29 |
| Trouvé: | C 65,93 | H 5,90 | N 9,95 |

Exemple 8

Méthoxyméthyl-1 oxo-4 dihydro-1,4 quinoléine N-cyclopentyl carboxamide-3.

(I: $R_1$ = 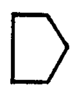 , $R_2$ = $CH_3$, n = 1, X = H) dérivé n° 8 (SR 26029).

F = 155°C (Rdt. 55%)
Analyse $C_{17}H_{20}N_2O_3$

| | | | |
|---|---|---|---|
| Calculé: | C 67,98 | H 6,71 | N 9,33 |
| Trouvé: | C 68,12 | H 6,73 | N 9,29 |

Exemple 9

Méthoxyméthyl-1 oxo-4 fluoro-6 dihydro-1,4 quinoléine N-propyl carboxamide-3 (I: $R_1 = C_3H_7$, $R_2 = CH_3$, N = 1, X = F); dérivé n° 9 (SR 26049).

F = 162°C (Rdt. 50%)
Analyse $C_{15}H_{17}N_2O_3$

| | | | |
|---|---|---|---|
| Calculé: | C 61,64 | H 5,86 | N 9,58 |
| Trouvé: | C 61,62 | H 5,91 | N 9,34 |

Ce composé a été préparé selon le mode opératoire décrit à l'exemple 1 à partir du Méthoxyméthyl-1 oxo-4 fluoro-6 dihydro-1,4 quinoléine carboxylate d'éthyle-3 (IV: $R_2 = CH_3$, n = 1, X = F; F = 170°C; Rdt. 50%)

Exemple 10

Méthoxyméthyl-1 oxo-4 fluoro-8 dihydro-1,4 quinoléine N-propyl carboxamide-3 (I: $R_1 = C_3H_7$, $R_2 = CH_3$, n = 1, X = F); dérivé n° 10 (SR 26109).

F = 134°C (Rdt. 67%)
Analyse $C_{15}H_{17}FN_2O_3$

| | | | |
|---|---|---|---|
| Calculé: | C 61,64 | H 5,86 | N 9,58 |
| Trouvé: | C 61,65 | H 5,68 | N 9,45 |

Ce composé a été préparé selon le mode opératoire décrit à l'exemple 1 à partir du méthoxyméthyl-1 oxo-4 fluoro-8 dihydro-1,4 quinoléine carboxylate d'éthyle-3 (IV: $R_2 = CH_3$, n = 1, X = F; F = 142°C; Rdt. 44%)

Exemple 11

Méthoxyméthyl-1 oxo-4 chloro-8 dihydro-1,4 quinoléine N-propyl carboxamide-3. (I: $R_1 = C_3H_7$, $R_2 = CH_3$, n = 1, X = Cl); dérivé n° 11 (SR 26117).

F = 152°C (Rdt. 40%)

Analyse $C_{15}H_{17}ClN_2O_3$

| | | | |
|---|---|---|---|
| Calculé: | C 58,35 | H 5,55 | N 9,07 |
| Trouvé: | C 58,52 | H 5,57 | N 8,97 |

Ce composé a été préparé selon le mode opératoire décrit à l'exemple 1 à partir du méthoxyméthyl-1 oxo-4 chloro-8 dihydro-1,4 quinoléine carboxylate d'éthyle-3 (IV: $R_2 = CH_3$, N = 1, X = Cl; F = 110°C; Rdt. 35%)

Exemple 12

Méthoxyméthyl-1 oxo-4 méthoxy-6 dihydro-1,4 quinoléine N-propyl carboxamide-3 (I: $R_1 = C_3H_7$, $R_2 = CH_3$, n = 1, X = $OCH_3$); dérivé n° 12 (SR 25896).

F = 200°C (Rdt. 50%)

Analyse $C_{16}H_{20}N_2O_4$

| | | | |
|---|---|---|---|
| Calculé: | C 63,14 | H 6,62 | N 9,21 |
| Trouvé: | C 63,02 | H 6,67 | N 9,14 |

Ce composé a été préparé selon le mode opératoire décrit à l'exemple 1 à partir du méthoxyméthyl-1 oxo-4 méthoxy-6 dihydro-1,4 quinoléine carboxylate d'éthyle-3 (IV: $R_2 = CH_3$, n = 1, X = $OCH_3$; F = 149°C, Rdt. 30%)

Exemple 13

Ethoxyméthyl-1 oxo-4 dihydro-1,4 quinoléine N-propyl carboxamide-3 (I: $R_1 = C_3H_7$, $R_2 = C_2H_5$, n = 1, X = H); dérivé n° 13 (SR 25983).

1°) Ethoxyméthyl-1 oxo-4 dihydro-1,4 quinoléine carboxylate d-éthyle-3 (IV: $R_2 = C_2H_5$, n = 1, X = H)

Un mélange d'hydroxy-4 quinoléine carboxylate d'éthyle-3 (4 g 0,016 mole) et de carbonate de potassium (3,5 g 0,025 mole) dans le diméthylformamide (40 ml) est porté pendant 30 minutes à 90°C. Après addition du chlorométhyléthyléther (1,93 ml, 0,021 mole) le chauffage est continué pendant 30 heures. Après filtration et évaporation du solvant le produit attendu est lavé à l'eau et séché: cristaux incolores; F = 64°C (Rdt. 89%).

2°) Ethoxyméthyl-1 oxo-4 dihydro-1,4 quinoléine N-propyl carboxamide-3

Le produit obtenu à l'étape précédente (2 g, 0,00726 mole) en solution dans la propylamine (60 ml) est porté 20 heures à 80°C. Le produit attendu cristallise à froid. Il est lavé à l'éther isopropylique et séché: cristaux incolores;) F = 150°C (Rdt. 80%).

Analyse $C_{16}H_{20}N_2O_3$

| | | | |
|---|---|---|---|
| Calculé: | C 66,65 | H 6,99 | N 9,72 |
| Trouvé: | C 66,27 | H 7,18 | N 9,52 |

Exemple 14

Ethoxyéthyl-1 oxo-4 dihydro-1,4 quinoléine N-propyl carboxamide-3 (I: $R_1 = C_3H_7$, $R_2 = C_2H_5$, n = 2, X = H); dérivé n° 14 (SR 26002)

1°) Ethoxyéthyl-1 oxo-4 dihydro-1,4 quinoléine carboxylate d'éthyle-3 (IV: $R_2 = C_2H_5$, n = 2, X = H)

Un mélange d'hydroxy-4 quinoléine carboxylate d'éthyle-3 (5 g 0,02 mole) et de carbonate de potassium (4,38 g 0,0308 mole) dans le diméthylformamide (60 ml) est porté pendant 30 minutes à 90°C. Après addition de bromoéthyléthyléther (2,9 ml, 0,026 mole) le chauffage est maintenu pendant 20 heures. Après filtration et évaporation du solvant, le produit attendu est lavé à l'eau: cristaux incolores, F = 107°C (Rdt. 96%)

2°) Ethoxyéthyl-1 oxo-4 dihydro-1,4 quinoléine N-propyl carboxamide-3

Un mélange du produit précédent (2 g, 0,0069 mole) et de propylamine (120 ml) est porté 48 heures à 80°C. Après évaporation le produit attendu est chromatographié sur colonne de silice avec l'acétate d'éthyle: cristaux incolores F = 116°C (Rdt. 45%).

Analyse $C_{17}H_{22}N_2O_3$

| | | | |
|---|---|---|---|
| Calculé: | C 67,53 | H 7,33 | N 9,26 |
| Trouvé: | C 67,62 | H 7,49 | N 9,11 |

Exemple 15

Méthoxyéthyl-1 oxo-4 dihydro-1,4 quinoléine N-propyl carboxamide-3 (I: $R_1 = C_3H_7$, $R_2 = CH_3$, n = 2, X = H); dérivé n° 15 (SR 26068).

F = 114°C (Rdt. 28%)

Ce composé a été préparé selon le mode opératoire décrit à l'exemple 14 à partir du méthoxyéthyl-1 oxo-4 dihydro-1,4 quinoléine carboxylate d'éthyl-3 (IV: $R_2 = CH_3$, n = 2, X = H; F = 140°C, Rdt. 40%).

Analyse $C_{16}H_{20}N_2O_3$

| | | | |
|---|---|---|---|
| Calculé: | C 66,64 | H 6,99 | N 9,72 |
| Trouvé: | C 66,72 | H 7,25 | N 9,80 |

Les résultats des essais toxicologiques et pharmacologiques qui sont rapportés ci-après, ont permis de mettre en évidence les intéressantes propriétés de l'invention, en particulier, des propriétés anticonvulsivante et psychotonique.

L'invention a donc pour objet un médicament ayant en particulier des activités anticonvulsivante et psychotonique caractérisé en ce qu'il contient à titre de principe actif, un dérivé de formule (I) ou un sel d'addition avec un acide minéral ou organique thérapeutiquement accpetable.

Etude toxicologique

Les composés de l'invention bénéficient d'une bonne tolérance et d'une faible toxicité. A titre indicatif, la DL 50 pour la voie orale est de 1100 mg/Kg pour le dérivé N°1, chez la souris.

Les essais effectués sur différentes espèces animales sur les toxicités aigue, subchronique et chronique, n'ont pas mis en évidence une quelconque réaction locale ou générale, perturbation ou anomalie dans les examens biochimiques, macroscopiques et microscopiques effectués tout au long des essais.

Etude pharmacologique

1) Activité anticonvulsivante

Cette activité à été étudiée par la test au pentetrazole, selon la méthode de EVERETT et RICHARDS (J. Pharm. Exp. Ther. 1944, *81*, 402—407). Le produit à tester est administré par voie orale à des lots de 10 souris (mâles CDI, 20—25 g) 30 minutes avant l'administration du pentétrazole par la voie sous-cutanée, à la dose de 135 mg/kg.

On note dans chaque lot le nombre d'animaux qui ne présentent pas de crise tonique pendant les 30 minutes qui suivent l'administration de l'agent convulsivant. On détermine ainsi la DE 50 qui est la dose qui évite l'apparition des crises chez la moitié des animaux.

Les DE 50 ont été calculées par la méthode de D. K. FINNEY (Probit analysis; University Press; Cambridge; 1971).

Les résultats sont rassemblés dans le tableau suivant:

| dérivé numéro | $DE_{50}$ (mg/Kg) |
|---|---|
| 1 | 55 |
| 5 | 46 |
| 7 | 67 |
| 8 | 108 |
| 9 | 62 |
| 10 | 53 |
| 11 | 21 |
| 12 | 65 |
| 13 | 75 |
| 14 | 79 |
| 15 | 50 |
| valproate de sodium | 200 |
| phénobarbital | 10 |

2) Activité locomotrice

Cette activité a été étudiée selon la méthode de J. BOISSIER (Arch. Int. Pharmacodyn., 1965, *158*, 212—221).

Les souris (mâles CDI 20—25 g) sont placées par groupe de trois dans les cages d'un actimètre à cellule photoélectrique. Après 30 minutes d'habituation à l'enceinte, les animaux sont traités par la voie orale avec le produit à tester en suspension dans la carboxyméthylcellulose à 1%. Un lot témoin de souris non traitées, reçoit uniquement le véhicule. Chaque lot comporte 12 groupes de 3 souris. Les réponses d'activité motrice sont enregistrées pendant 80 minutes.

Les résultats sont rassemblés dans le tableau suivant:

| | Doses mg/Kg V.O. | Activité motrice pendant 80 mn | P (Test de student) |
|---|---|---|---|
| Souris non traitées | 0 | 553 ± 200 | |
| Souris traitées par le dérivé N°1 | 32 | 1842 ± 470 | <0,05 |
| Souris non traitées | 0 | 1340 ± 159 | |
| Souris traitées par le dérivé n°3 | 32 | 3070 ± 219 | < 0,05 |

3) Activité antagoniste vis-à-vis des effets hypnotiques des benzodiazépines

Cette activité a été étudiée selon la méthode de J. JANSSEN (J. Med. Pharm. Chem., 1959, *1*, 281—297).

Les produits de l'invention, placés en suspension dans la carboxyméthylcellulose à 1% ont été administrés par voie intrapéritonéale à des lots de 10 souris (mâles $CD_1$, 20—25 g). La benzodiazépine (diazépam) a été administrée par voie i.p. 15 minutes après les composés de l'invention. Un groupe témoin a reçu la carboxyméthylcellulose avant le diazépam. On a jugé l'effet hypnotique en notant dans chaque lot, le temps pendant lequel les animaux ont perdu le reflexe de redressement.

RESULTATS

| Dérivé SR | Dose (mg/kg i.p.) | Diazépam Dose (mg/kg i.p.) | Durée du sommeil en mn. |
|---|---|---|---|
| témoin | 0 | 6 | 97 ± 12 |
| dérivé n° 1 | 25 | 6 | 39 ± 20* |
| dérivé n° 7 | 25 | 6 | 32 ± 11* |
| dérivé n° 9 | 25 | 6 | 22 ± 8* |
| dérivé n° 3 | 25 | 6 | 9 ± 4* |
| dérivé n° 5 | 25 | 6 | 62 ± 10* |

* P< 0,05 par rapport au lot témoin: test "t" de Student.

Les études toxicologique et pharmacologique qui viennent d'être rapportées ont mis en évidence la faible toxicité des composés de l'invention et leur bonne tolérance, ainsi que leurs interessantes propriétés qui les rendent très utiles en thérapeutique humaine et vétérinaire, et justifient leur utilisation à titre de médicament.

Le médicament de l'invention peut être présenté pour l'administration orale sous forme de comprimés, comprimés dragéifiés, capsules, gouttes, granulés ou sirop.

Il peut être aussi présenté pour l'administration rectale sous forme de suppositoires et pour l'administration parentérale sous forme de soluté injectable.

Chaque dose unitaire contient avantageusement de 5 mg à 300 mg de principe actif, les doses administrables journellement pouvant varier de 5 mg à 300 mg de principe actif en fonction de l'âge du patient et de la sévérité de l'affection traitée.

On donnera ci-après quelques formulations pharmaceutiques du médicament de l'invention:

| | |
|---|---|
| 1) Comprimés | |
| dérivé N° 1 | 0,030 g |
| excipient | amidon de blé, lactose, silice colloidale, talc, stéarate de magnésium. |
| 2; Comprimés dragéifiés | |
| dérivé N° 11 | 0,015 g |
| excipient | polyvinyl pyrrolidone, carboxyméthylcellulose sodique, stéarate de magnésium, levilite, hydroxypropilméthylcellulose, oxyde de titane, cire blanche. |
| 3) Capsules | |
| dérivé N° 3 | 0,100 g |
| excipient | talc, lactose, stéarate de magnésium. |
| 4) Suppositioires | |
| dérivé N° 10 | 0,050 g |
| excipient | triglycérides semi-synthétiques |
| 5) Soluté injectable | |
| dérivé N° 15 | 0,025 g |
| excipient | solvant isotonique q.s.p. 3 ml |

Possédant d'intéressantes propriétés anticonvulsivante et psychotonique et doué d'une bonne tolérance, le médicament de l'invention est indiqué aussi bien chez l'adulte que chez l'enfant dans le traitement des convulsions, des asthénies fonctionnelles, des troubles de la mémoire, de l'attention et aussi comme agent facilitant le réveil chez le malade anesthésié par des benzodiazépines.

**Revendications pour les Etats contractants: BE CH DE FR GB IT LI LU NL SE**

1. Composés de formule

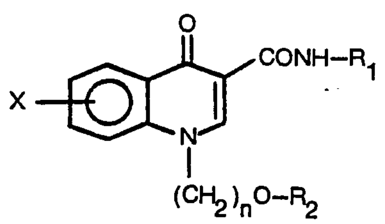

(I)

dans laquelle:

$R_1$ représente un radical alcoyle inférieur linéaire ou ramifié comprenant de 1 à 5 atomes de carbone; un radical alcényle ou alcynyle de 2 à 5 atomes de carbone; ou un radical cycloalcoyle comprenant de 3 à 5 atomes de carbone,

$R_2$ représente un radical alcoyle comprenant de 1 à 4 atomes de carbone, n est égal à 1 ou 2,

X représente un hydrogène, un halogène, un groupe alcoxy ou alcoyle de 1 à 4 atomes de carbone.

2. Méthoxyméthyl-1 oxo-4 dihydro-1,4 quinoléine N-propyl carboxamide-3 et ses sels d'acide minéraux ou organiques pharmaceutiquement acceptables.

3. Méthoxyméthyl-1 oxo-4 dihydro-1,4 quinoléine N-éthyl carboxamide-3 et ses sels d'acides minéraux et organiques pharmaceutiquement acceptables.

4. Méthoxyméthyl-1 oxo-4 dihydro-1,4 quinoléine N-isobutyl carboxamide-3 et ses sels d'acides minéraux oetorganiques pharmaceutiquement acceptables.

5. Méthoxyméthyl-1 oxo-4 fluoro-6 dihydro-1,4 quinoléine N-propyl carboxamide-3 et ses sels d'acides minéraux et organiques pharmaceutiquement acceptables.

6. Procédé de préparation des composés de formule (I) caractérisé en ce que les composés de formule

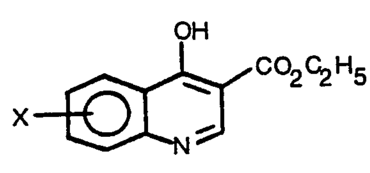

(II)

dans laquelle X a les mêmes significations que dans la formule (I) sont mis à réagir avec un dérivé halogéné de formule

$$Y—(CH_2)_n—O—R_2 \qquad (III)$$

dans lequel Y représente un halogène tel que le chlore ou le brome et n et $R_2$ ont les mêmes valeurs que dans la formule (I) pour obtenir les composés de formule (IV).

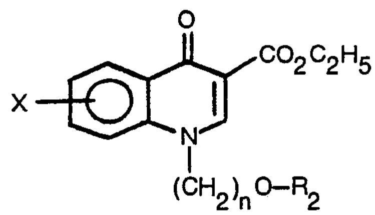

(IV)

qui sont mis à réagir avec une amine de formule

$$H_2N—R_1 \qquad (V)$$

dans laquelle $R_1$ a les mêmes significations que dans la formule (I) pour obtenir le composés de formule (I).

7. Procédé selon la revendication 6 caractérisé en ce que la préparation des composés de formule (III) est effectuée par chauffage à une température comprise entre 60°C et 130°C en présence d'une base telle que le carbonate de potassium dans un solvant organique tel que le diméthylformamide.

8. Procédé selon l'une des revendications 6 ou 7 caractérisé en ce que la fixation du radical aminé de formule (V) est effectuée par chauffage des deux réactifs en présence d'un excès de l'amine (V) à une température comprise entre 60°C et 150°C pendant une durée de 10 h. à 48 h.

9. Médicament caractérisé en ce qu'il contient, à titre de principe actif, un dérivé suivant l'une quelconque des revendications 1 à 5 ou l'un de ses sels pharmaceutiquement acceptables.

10. Médicament selon la revendication 9, caractérisé en ce que chaque dose unitaire contient de 0,05 g à 0,300 g de principe actif.

**Revendications pour les l'Etat contractants: AT GR ES**

1. Procédé de préparation de composés de formule

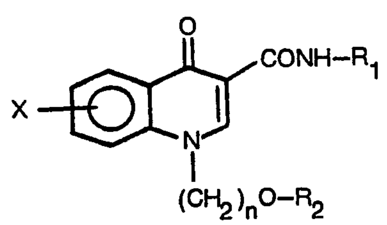

(I)

dans laquelle:

$R_1$ représente un radical alcoyle inférieur linéaire ou ramifié comprenant de 1 à 5 atomes de carbone; un radical alcényle ou alcynyle de 2 à 5 atomes de carbone; ou un radical cycloalcoyle comprenant de 3 à 5 atomes de carbone,

$R_2$ représente un radical alcoyle comprenant de 1 à 4 atomes de carbone, n est égal à 1 ou 2,

X représente un hydrogène, un halogène, un groupe alcoxy ou alcoyle de 1 à 4 atomes de carbone, procédé caractérisé en ce que les composés de formule

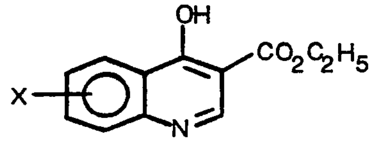

(II)

dans laquelle X a les mêmes significations que dans la formule (I) sont mis à réagir avec un dérivé halogéné de formule

$$Y—(CH_2)_n—O—R_2 \qquad (III)$$

dans lequel Y représente un halogène tel que le chlore ou le brome et n et $R_2$ ont les mêmes valeurs que dans la formule (I) pour obtenir les composés de formule (IV)

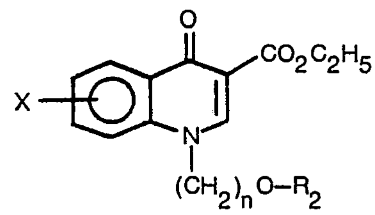

(IV)

qui sont mis à réagir avec une amine de formule

$$H_2N—R_1 \qquad (V)$$

dans laquelle $R_1$ a les mêmes significations que dans la formule (I) pour obtenir le composés de formule (I).

2. Procédé selon la revendication 1, caractérisé en ce que la préparation des composés de formule (III) est effectuée par chauffage à une température comprise entre 60°C et 130°C en présence d'une base telle que le carbonate de potassium dans un solvant organique tel que le diméthylformamide.

3. Procédé selon l'une des revendications 1 ou 2, caractérisé en ce que la fixation du radical aminé de formule (V) est effectuée par chauffage des deux réactifs en présence d'un excès de l'amine (V) à une température comprise entre 60°C et 150°C pendant une durée de 10 heures à 48 heures.

4. Procédé de préparation d'un médicament, caractérisé en ce qu'on utilise, à titre de principe actif, un dérivé obtenu suivant l'une quelconque des revendications 1 à 3 ou l'un de ses sels pharmaceutiquement acceptables.

**Patentansprüche für die Vertragsstaaten: BE CH DE FR GB IT LI LU NL SE**

1. Verbindungen der Formel (I),

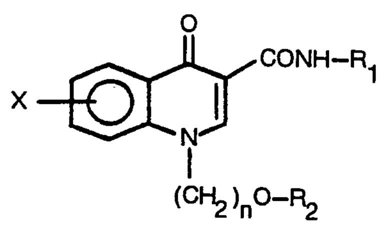

(I)

in der bedeuten:

$R_1$ einen geradkettigen oder verzweigten niedrigen Alkylrest mit 1 bis 5 Kohlenstoffatomen, einen Alkenyl- oder Alkinylrest mit 2 bis 5 Kohlenstoffatomen oder einen Cycloalkylrest mit 3 bis 5 Kohlenstoffatomen,

$R_2$ einen Alkylrest mit 1 bis 4 Kohlenstoffatomen,

n 1 oder 2,

X Wasserstoff, Halogen, einen Alkoxy- oder Alkylrest mit 1 bis 4 Kohlenstoffatomen.

2. 1-Methoxymethyl-4-oxo-1,4-dihydrochinolin-3-N-propylcarboxamid und seine anorganischen oder organischen, pharmazeutisch verträglichen Säuresalze.

3. 1-Methoxymethyl-4-oxo-1,4-dihydrochinolin-3-N-ethylcarboxamid und seine anorganischen und organischen, pharmazeutisch verträglichen Säuresalze.

4. 1-Methoxymethyl-4-oxo-1,4-dihydrochinolin-3-N-isobutylcarboxamid und seine anorganischen und organischen, pharmazeutisch verträglichen Säuresalze.

5. 1-Methoxymethyl-4-oxo-6-fluor-1,4-dihydrochinolin-3-N-propylcarboxamid und seine anorganischen und organischen, pharmazeutisch verträglichen Säuresalze.

6. Verfahren zur Herstellung der Verbindungen der Formel (I), dadurch gekennzeichnet, daß Verbindungen der Formel (II),

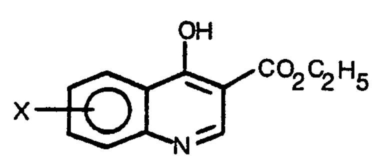

(II)

in der X die in Formel (I) angegebene Bedeutung hat, mit einem Halogenderivat der Formel (III),

$$Y{-}(CH_2)_n{-}O{-}R_2 \qquad (III)$$

in der Y Halogen, wie Chlor oder Brom, ist und n und $R_2$ die in Formel (I) angegebene Bedeutung haben, zu Verbindungen der Formel (IV) umgesetzt werden,

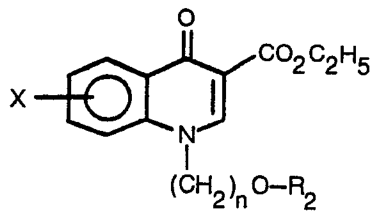

(IV)

die wiederum mit einem Amin der Formel (V),

$$H_2N{-}R_1 \qquad (V)$$

in der $R_1$ die in Formel (I) angegebene Bedeutung hat, zu Verbindungen der Formel (I) umgesetzt werden.

7. Verfahren nach Anspruch 6, dadurch gekennzeichnet, daß die Herstellung der Verbindungen der Formel (III) durch Erhitzen auf eine Temperatur von 60 bis 130°C in Gegenwart einer Base, wie Kaliumcarbonat, in einem organischen Lösungsmittel, wie Dimethylformamid, durchgeführt wird.

8. Verfahren nach Anspruch 6 oder 7, dadurch gekennzeichnet, daß die Umsetzung des Aminrestes der Formel (V) durch Erhitzen der beiden Bestandteile in Gegenwart eines Überschusses des Amins (V) auf eine Temperatur von 60 bis 150°C während 10 bis 48 h erfolgt.

9. Arzneimittel, gekennzeichnet durch den Gehalt an einem Derivat nach einem der Ansprüche 1 bis 5 oder einem seiner pharmazeutisch verträglichen Salze als Wirkstoff.

10. Arzneimittel nach Anspruch 8, dadurch gekennzeichnet, daß jede Einzeldosis von 0,005 bis 0,300 g Wirkstoff enthält.

**Patentansprüche für die Vertragsstaaten: AT GR ES**

1. Verfahren zur Herstellung von Verbindungen der Formel (I),

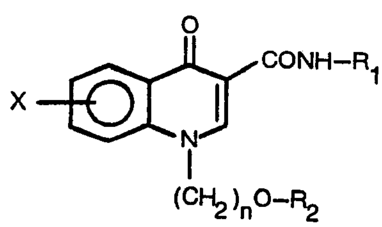

(I)

in der bedeuten:

$R_1$ einen geradkettigen oder verzweigten niedrigen Alkylrest mit 1 bis 5 Kohlenstoffatomen, einen Alkenyl- oder Alkinylrest mit 2 bis 5 Kohlenstoffatomen oder einen Cycloalkylrest mit 3 bis 5 Kohlenstoffatomen,

$R_2$ einen Alkylrest mit 1 bis 4 Kohlenstoffatomen,

n 1 oder 2,

X Wasserstoff, Halogen, einen Alkoxy- oder Alkylrest mit 1 bis 4 Kohlenstoffatomen, dadurch gekennzeichnet, daß Verbindungen der Formel (II),

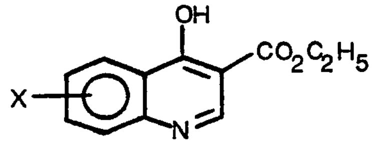

(II)

in der X die in Formel (I) angegebene Bedeutung hat, mit einem Halogenderivat der Formel (III),

$$Y{-}(CH_2)_n{-}O{-}R_2 \qquad (III)$$

in der Y ein Halogen, wie Chlor oder Brom, ist und n und $R_2$ die in Formel (I) angebene Bedeutung haben, zu Verbindungen der Formel (IV) umgesetzt werden,

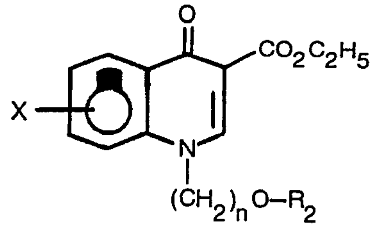

(IV)

die wiederum mit einem Amin der Formel (V)

$$H_2N—R_1 \qquad (V)$$

in der $R_1$ die in Formel (I) angegebene Bedeutung hat, zu Verbindungen der Formel (I) umgesetzt werden.

2. Verfahren nach Anspruch 1, dadurch gekennzeichnet, daß die Herstellung der Verbindungen der Formel (III) durch Erhitzen auf eine Temperatur von 60 bis 130°C in Gegenwart einer Base, wie Kaliumcarbonat, in einem organischen Lösungsmittel, wie Dimethylformamid, durchgeführt wird.

3. Verfahren nach Anspruch 1 oder 2, dadurch gekennzeichnet, daß die Umsetzung des Aminrestes der Formel (V) durch Erhitzen der beiden Bestandteile in Gegenwart eines Überschusses des Amins (V) auf eine Temperatur von 60 bis 150°C während 10 bis 48 h erfolgt.

4. Verfahren zur Herstellung eines Arzneimittels, gekennzeichnet durch Verwendung eines nach einem der Ansprüche 1—3 erhaltenen Derivats oder eines seiner pharmazeutisch verträglichen Salze als Wirkstoff.

**Claims for the Contracting States: BE CH DE FR GB IT LI LU NL SE**

1. Compounds of formula

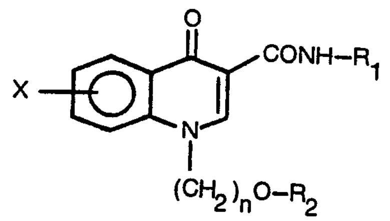

(I)

wherein:

$R_1$ represents a straight-chained or branched lower alkyl radical containing from 1 to 5 carbon atoms; an alkenyl or alkynyl radical containing from 2 to 5 carbon atoms; or a cycloalkyl radical containing from 3 to 5 carbon atoms,

$R_2$ represents an alkyl radical containing from 1 to 4 carbon atoms;

n is equal to 1 or 2,

X represents a hydrogen, a halogen, an alkoxy or alkyl group with 1 to 4 carbon atoms.

2. 1-methoxy-4-oxo-1,4-dihydro-quinoline-3-(N-propyl)-carboxamide and the pharmaceutically acceptable inorganic or organic acid addition salts thereof.

3. 1-methoxy-4-oxo-1,4-dihydro-quinoline-3-(N-ethyl)-carboxamide and the pharmaceutically acceptable inorganic or organic acid addition salts thereof.

4. 1-methoxy-4-oxo-1,4-dihydro-quinoline-3-(N-isobutyl)-carboxamide and the pharmaceutically acceptable inorganic or organic acid addition salts thereof.

5. 1-methoxy-4-oxo-6-fluoro--1,4-dihydro-quinoline-3-(N-propyl)-carboxamide and the pharmaceutically acceptable inorganic or organic acid addition salts thereof.

6. Process for preparing compounds of formula (I), characterized in that the compounds of formula

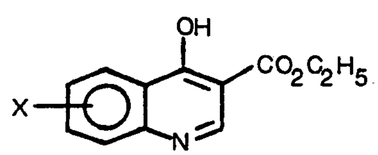

(II)

wherein X has the same meanings as in formula (I), are reacted with a halogenated derivative of formula

$$Y—(CH_2)_n—O—R_2 \qquad (III)$$

wherein Y represents a halogen such as chlorine or bromine and n and $R_2$ have the same values as in formula (I), so as to obtain the compounds of formula (IV)

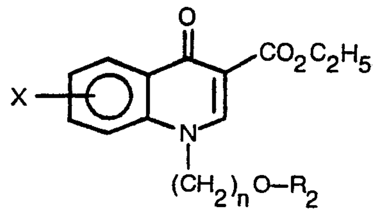

(IV)

which are reacted with an amine of formula

$$H_2N—R_1 \qquad (V)$$

wherein $R_1$ has the same meanings as in formula (I), so as to obtain the compounds of formula (I).

7. Process according to claim 6, characterized in that compounds of formula (III) are prepared by heating to a temperature between 60° and 130° in the presence of a base such as potassium carbonate in an organic solvent such as dimethylformamide.

8. Process according to one of claims 6 or 7, characterized in that the amino radical of formula (V) is fixed by heating the two reagents in the presence of an excess of the amine (V) to a temperature between 60° and 150° for a period of 10 hours to 48 hours.

9. Pharmaceutical composition, characterized in that it contains, as active principle, a derivative according to any one of claims 1 to 5 or one of the pharmaceutically acceptable salts thereof.

10. Pharmaceutical composition according to claim 9, characterized in that each dosage unit contains from 0.05 g to 0.300 g of active principle.

**Claims for the Contracting States: AT GR ES**

1. Process for preparing compounds of formula

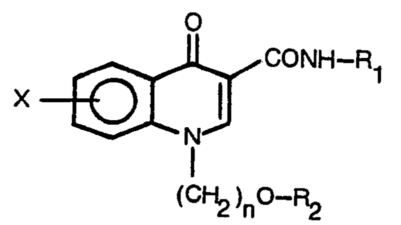

(I)

wherein:

$R_1$ represents a straight-chained or branched lower alkyl radical containing from 1 to 5 carbon atoms; an alkenyl or alkynyl radical containing from 2 to 5 carbon atoms; or a cycloalkyl radical containing from 3 to 5 carbon atoms,

$R_2$ represents an alkyl radical containing from 1 to 4 carbon atoms;

n is equal to 1 or 2,

X represents a hydrogen, a halogen, an alkoxy or alkyl group with 1 to 4 carbon atoms, characterized in that the compounds of formula

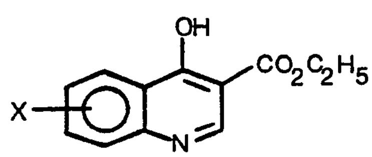

(II)

wherein X is defined as in formula (I), are reacted with a halogenated derivative of formula

$$Y—(CH_2)_n—O—R_2 \qquad (III)$$

wherein Y represents a halogen such as chlorine or bromine and n and $R_2$ have the same values as in formula (I), so as to obtain the compounds of formula (IV)

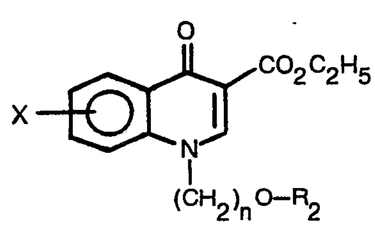

(IV)

which are reacted with an amine of formula

$$H_2N—R_1 \qquad (V)$$

wherein $R_1$ is defined as in formula (I), to obtain the compounds of formula (I).

2. Process according to claim 1, characterized in that the compounds of formula (III) are prepared by heating to a temperature between 60° and 130° in the presence of a base such as potassium carbonate in an organic solvent such as dimethylformamide.

3. Process according to one of claims 1 or 2, characterized in that the amine radical of formula (V) is fixed by heating the two reagents in the presence of an excess of the amine (V) to a temperature between 60° and 150° for a period of from 10 hours to 48 hours.

4. Process for preparing a pharmaceutical composition, characterized in that a derivative obtained according to any one of claims 1 to 3 or one of the pharmaceutically acceptable salts thereof is used as active principle.